# EUROPEAN PATENT APPLICATION

(11) **EP 3 000 478 A1**
(43) Date of publication of application: **30.03.2016**
(21) Application number: 14801454.1
(22) Date of filing: 23.05.2014
(51) Int. Cl.: A61K 39/395, A61P 31/12, A61P 31/18

(54) **THERAPEUTIC OR PROPHYLACTIC AGENT FOR IMMUNODEFICIENCY VIRUS INFECTION**

(30) Priority: 23.05.2013 JP 2013108772
(71) Applicant: IDAC Theranostics, Inc., Bunkyo-ku Tokyo 113-0033 (JP)
(72) Inventor: ITO, Satoru, Tokyo 113-0033 (JP); YOKOCHI, Shoji, Tokyo 113-0033 (JP)
(74) Representative: Keirstead, Tanis Evelyne
(86) International application number: PCT/JP2014/063732
(87) International publication number: WO 2014/189138

(57) **Abstract**

Disclosed is a novel means that enables complete cure of not only acute and chronic virus infections, but also latent retrovirus infections in which incorporation of the virus into the host chromosome has occurred. In the present invention, by destroying cells containing molecules that act as scaffolds for the virus infection in the patient's body, cells *per se* that have been already infected with virus are destroyed while inhibiting expansion of the virus infection in the patient's body, so that final complete cure of virus infection can be realized. The treatment of immunodeficiency virus infection may be carried out by administering an antibody or the like having high cytotoxic activity to the patient to destroy at least any of CD4 molecule-containing cells, CCR5 molecule-containing cells, and CXCR4 molecule-containing cells, preferably CD4 molecule-containing cells.

## Description

### TECHNICAL FIELD

The present invention relates to a therapeutic or prophylactic agent for infection with a virus such as human immunodeficiency virus.

### BACKGROUND ART

Infection with HIV virus has been feared as an infection which causes acquired immunodeficiency syndrome (the so-called AIDS), finally leading to death. However, due to efforts by a number of people, inhibitors for the respective steps in the life cycle of the virus have been developed as a result of research on the viral life cycle. In particular, introduction of a method in which several steps are simultaneously inhibited (cART) enabled control of development of AIDS, although complete cure of the infection is still impossible. That is, now it is possible to keep the state where RNA virus is not detected in blood (Non-patent Documents 1 and 2). However, it has been found, in fact, that the virus is present in lymph nodes and enteric tissues (Non-patent Documents 3 and 4). Since the virus is a retrovirus, the virus is mostly present in a state where the virus is incorporated in the chromosome of the host cells and sleeping, so that the complete cure has been considered to be impossible. Thus, there are problems such as an enormous drug cost during lifetime, acquisition of drug resistance due to missed doses, and side effects of the drugs. Accordingly, the complete cure has been increasingly demanded in recent years (Non-patent Document 5).

In a case of a patient with HIV infection who lives in Berlin, the patient, who had been receiving polypharmacy (ART), also developed a blood cancer (AML). First, the anti-HIV prescription (ART) was stopped and treatment of AML was carried out intensively. As a result, AML was ameliorated, but the interruption of ART allowed explosive growth of HIV virus. The ART polypharmacy was then carried out again, resulting in successful control of HIV and recovery of stable conditions. However, since recurrence of AML occurred, treatment was carried out by bone marrow transplantation from a patient having CCR5 deficiency as a last-ditch measure (Non-patent Document 6). Here, an explanation will be given for the CCR5 deficiency. This molecule is one of the receptor molecules used by HIV virus upon its infection. That is, the CCR5 molecule is one of the molecules that act as scaffolds for HIV infection. The main scaffold for the infection is the CD4 molecule, and establishment of the infection requires association of CCR5 with CD4. It has been revealed that there are patients who do not develop AIDS after the infection because of their genetic background. They have been shown to have genetic deficiency in the CCR5 molecule (Non-patent Document 7). This is the reason why the bone marrow from the patient with CCR5 deficiency was used for the bone marrow transplantation. The ART/HIV polypharmacy has not been carried out since then, but the state where HIV virus is not detected in blood has continued for not less than 4 years after the bone marrow transplantation, leading to clinical remission. This case is called "miracle of Berlin".

However, since some HIV viruses use another receptor molecule CXCR4 as a scaffold instead of CCR5, there remains a problem in cases where only transplantation of bone marrow with CCR5 deficiency is carried out. There is little information on the relationship between the polymorphic genetic state of CXCR4 in human and the symptom onset. Moreover, when the transplantation is to be considered, the hurdle of HLA matching needs to be overcome. Hearing this news, many patients wished to receive bone marrow transplantation, but it was found that the transplantation was possible only for several combinations in the world. There is a report on a therapy which was carried out in view of this, wherein hematopoietic stem cells were collected from a patient infected with HIV who also developed AML, and the cells were then genetically manipulated such that they were incapable of expressing CCR5 molecules, followed by returning the resulting cells into the patient. This therapy was shown to be effective, although it has been carried out only for several cases in each of several facilities. However, these are obviously special cases (Non-patent Document 8). Although this gene manipulation method may enable treatment for either the CCR5-type virus or the CXCR4-type virus, these cases should be considered to be very special cases. This is because, in these cases, the HIV patients also developed the blood cancer, AML. In general infected patients, such gene manipulation requires the techniques of autologous blood collection, CD34-cell separation, gene manipulation, culturing, and transfer, so that the therapeutic method is too laborious and very costly. Although a technique utilizing CD34 cells from another individual should be considered, it cannot be easily carried out since the technique has a high risk of development of GVHD, a serious side effect accompanying bone marrow transplantation.

Now, let us again consider what the problems are in ART, which currently enables keeping of the state where free HIV virus is not detected in blood. There are the following problems: the health care cost is enormous because of life-long dosing, acquisition of drug resistance due to missed doses, which leads to limitation of prescribable drugs, and requirement of additional treatment due to occurrence of side effects of the drugs. All of these are due to activation of latent virus under certain conditions. In view of this, "purge & shock therapy" has been attempted, in which latent HIV virus is intentionally activated to force production of RNA virus particles, followed by performing polypharmacy. However, while control of the virus was successful *in vitro,* it was difficult *in vivo* (Non-patent Document 9). It was also revealed that the production of RNA virus particles does not lead to destruction of the cells infected therewith (Non-patent Document 10), and the fact that complete cure by this therapy is considerably difficult is now becoming obvious.

In terms of therapeutic methods for prevention of expansion of the infection, a drug named T-20, which targets CD4 molecules, and a drug named maraviroc, which targets CCR5, have already been approved and used for treatment. However, maraviroc can be prescribed only after confirming that the virus uses CCR5, and the test for the confirmation takes one month and is costly. Thus, maraviroc is not frequently prescribed. There are no drugs approved as inhibitors for the CXCR4-type virus. However, a number of reports have already been made showing emergence of strains resistant to these drugs.

As described above, therapeutic methods for keeping the blood level of HIV virus at less than the detection sensitivity using drugs that suppress the growth of HIV virus have been established so far, but elimination of the virus or the complete cure has not been achieved. In consideration of the idea that development of AIDS is linked to a decrease in the number of CD4 cells, a regimen in which ART is prescribed based on the criterion of the CD4 cell count of from 200 cells/ml to 500 cells/ml has been recommended (Non-patent Document 2). That is, conventional therapeutic methods are based on the idea that CD4-expressing cells should be kept as many as possible. For a lot of patients, the economic burden of the drugs which should be taken throughout the life to prevent development of AIDS is unbearable. Moreover, missing of doses quickly causes drug resistance, leading to limitation of available drugs. Furthermore, there are a number of patients suffering from side effects of the prescribed drugs themselves. Complete cure of HIV infection could not be achieved in any of such cases.

### PRIOR ART DOCUMENTS

### Non-patent Documents

Non-patent Document 1: A. R. Zolopa, The evolution of HIV treatment guidelines: current state-of-the-art of ART, Antiviral Res2010; 85: 241-244.
Non-patent Document 2: M. A. Thompson et al., Antiretroviral treatment of adult HIV infection: 2012 recommendations of the International Antiviral Society-USA panel, JAMA 2012; 308: 387-402.
Non-patent Document 3: M. Zeng et al., Cumulative mechanisms of lymphoid tissue fibrosis and T cell depletion in HIV-1 and SIV infections, J Clin Invest 2011; 121: 998-1008.
Non-patent Document 4: J. Cohen, HIV/AIDS research. Tissue says blood is misleading, confusing HIV cure efforts, Science 2011; 334: 1614.
Non-patent Document 5: D. Trono et al., HIV persistence and the prospect of long-term drug-free remissions for HIV-infected individuals, Science 2010; 329: 174-180.
Non-patent Document 6: NG. Hutter et al., Long-term control of HIV by CCR5 Delta32/Delta32 stem-cell transplantation, New Engl J Med 2009; 360: 692-698.
Non-patent Document 7: M. Dean et al., Genetic restriction of HIV-1 infection and progression to AIDS by a deletion allele of the CKR5 structural gene. Hemophilia Growth and Development Study, Multicenter AIDS Cohort Study, Multicenter Hemophilia Cohort Study, San Francisco City Cohort, ALIVE Study, Science 1996; 273: 1856-1862.
Non-patent Document 8: D. L. DiGiusto et al., RNA-based gene therapy for HIV with lentiviral vector-modified CD34(+) cells in patients undergoing transplantation for AIDS-related lymphoma, Sci Transl Med 2010; 2: 36ra43.
Non-patent Document 9: S. G. Deeks, HIV: Shock and kill, Nature 2012; 487: 439-440.
Non-patent Document 10: L. Shan et al., Stimulation of HIV-1-specific cytolytic T lymphocytes facilitates elimination of latent viral reservoir after virus reactivation, Immunity 2012; 36: 491-501.

### SUMMARY OF THE INVENTION

### PROBLEMS TO BE SOLVED BY THE INVENTION

Accordingly, an object of the present invention is to provide a novel means that enables complete cure of not only acute and chronic virus infections, but also latent retrovirus infections in which incorporation of the virus into the host chromosome has occurred.

### MEANS FOR SOLVING THE PROBLEMS

The present inventors focused attention on the fact that the case of the above-described miracle of Berlin utilized the idea of destroying the CCR5 molecule itself rather than inhibiting infection via this molecule. In HIV infection, CD4-positive cells are infected. Therefore, targeting of cells having CD4 molecules allows destroying of not only infected cells, but also uninfected cells having CD4 molecules. Conversely, in the absence of the molecule used as a scaffold for the infection, further infection is not established even if the virus is released in the living body due to destruction of cells, and thus extinction of the virus inevitably occurs. It can be understood that this is the situation experienced by the Berlin patient. As a result of intensive study based on such an understanding, the present inventors discovered that, by temporarily eliminating cells having CD4 molecules and/or the like used as a scaffold(s) for HIV infection from the body of a patient with HIV infection, expansion of the HIV infection in the body of the patient can be prevented and the virus infection can be treated even if the patient has latent infection with the virus incorporated in the chromosome, thereby completing the present invention.

That is, the present invention provides a therapeutic or prophylactic agent for immunodeficiency virus infection, comprising as an effective ingredient a substance that destroys at least any of CD4 molecule-containing cells, CCR5 molecule-containing cells, and CXCR4 molecule-containing cells. The present invention also provides a method for treatment or prevention of virus infection in a subject, said method comprising destroying cells which are present in the body of said subject and contain a molecule that acts as a scaffold for the virus infection.

### EFFECT OF THE INVENTION

According to the present invention, complete cure of HIV can be expected not only for cases of acute and chronic infections, but also for cases of latent retrovirus infections in which the virus is incorporated in the host chromosome, so that the patient can be free from the burden of continuing to take antiviral drugs throughout the life. Since it has been understood that development of AIDS after the virus infection is caused by a decrease in the number of CD4 cells, no one has positively attempted to destroy CD4 cells. The present invention aims to achieve complete cure of the virus infection based on an idea opposite to such conventional understanding.

### BRIEF DESCRIPTION OF THE DRAWINGS

Fig. 1 shows the results of measurement of the ADCC activity of the anti-CD4 humanized antibody IT1208 against CD4-containing cells among human peripheral blood mononuclear cells by flow cytometry analysis.
Fig. 2 shows the results of measurement of the ADCC activity of the anti-CD4 humanized antibody IT1208 against CD4-positive cells among human peripheral blood mononuclear cells using a commercially available assay kit.
Fig. 3 shows the results obtained by administering the anti-CD4 humanized antibody IT1208 to a monkey at 40 mg/kg (by 4 times of intravenous injection at one-week intervals) and measuring the number of CD4-containing T cells in venous blood (blood), rectum (rectum), and lymph nodes (LN). The ordinate represents the frequency (%) of CD4-containing cells, and the abscissa represents the number of days after the initial administration of IT1208.

### MODE FOR CARRYING OUT THE INVENTION

In the present invention, by destroying cells containing molecules that act as scaffolds for the virus infection in the patient's body, cells *per se* that have been already infected with virus are destroyed while inhibiting expansion of the virus infection in the patient's body, so that final complete cure of virus infection can be realized. If the virus loses its scaffolds for infection, new infection (acute infection) cannot be established, and invasion of the virus into uninfected cells is impossible even in the body of a patient who has been already infected, so that the growth of the virus in the body is suppressed, finally resulting in elimination of the virus from the body. By this method, all cells having the scaffold molecules are destroyed. Therefore, even in cases of latent retrovirus infection where the infecting virus is incorporated in the chromosome, latently-infected cells *per se* can be destroyed, and the growth of the virus can be prevented since the extracellularly released virus loses its place of infection. This enables complete cure of the virus infection as a result. Thus, the present invention is effective not only for acute and chronic infections of various viruses, but also for cases of retroviruses.

Immunodeficiency viruses such as human immunodeficiency virus and simian immunodeficiency virus use the CD4 molecule as a main scaffold for the infection, and also use the CCR5 molecule or the CXCR4 molecule for invasion into the cell. Therefore, by destroying at least any of CD4 molecule-containing cells, CCR5 molecule-containing cells, and CXCR4 molecule-containing cells in the patient's body to eliminate scaffolds for infection by the immunodeficiency virus, virus-infected cells *per se* can be destroyed, while inhibiting virus infection of uninfected cells. Thus, the therapeutic or prophylactic agent for immunodeficiency virus infection according to the present invention contains as an effective ingredient a substance that destroys at least any of CD4 molecule-containing cells, CCR5 molecule-containing cells, and CXCR4 molecule-containing cells. The agent may contain a plurality of such substances. The virus infection can be treated by destroying CCR5 molecule-containing cells in cases where the virus in the patient is of a CCR5-utilizing type, or by destroying CXCR4 molecule-containing cells in cases where the virus in the patient is of a CXCR4-utilizing type. However, the test for investigating whether the virus is of the CCR5 type or of the CXCR4 type requires time and cost (it is noted that whichever type of the immunodeficiency virus can be treated by administration of both a substance that destroys CCR5 molecule-containing cells and a substance that destroys CXCR4 molecule-containing cells). On the other hand, in cases of CD4 molecules, both types of immunodeficiency viruses use them as a scaffold, so that the type of the virus does not need to be investigated. Thus, in the present invention, it is preferred to cause depletion of CD4 molecule-containing cells in the patient's body, and the therapeutic or prophylactic agent of the present invention preferably contains a substance that specifically destroys CD4 molecule-containing cells.

As a means for specifically destroying cells containing a specific molecule present in the patient's body, an antibody against the molecule (more specifically, an antibody that recognizes the extracellular domain of the molecule) can be preferably used. An anti-CD4 antibody may be used for destroying CD4 molecule-containing cells; an anti-CCR5 antibody may be used for destroying CCR5 molecule-containing cells; or an anti-CXCR4 antibody may be used for destroying CXCR4 molecule-containing cells. It is known that antibodies have cytotoxic activities such as the ADCC (antibody-dependent cell-mediated cytotoxicity) activity and the CDC (complement-dependent cytotoxicity) activity. In the present invention, antibodies in which such activities are enhanced may be preferably used as means for destroying the cells. Either the ADCC activity or the CDC activity may be employed without a problem. The stronger the cytotoxic activity, the smaller the amount of the antibody used can be, so that the burden on the patient's body and the risk of side effects can be reduced, as well as higher economic advantage can be obtained. Thus, it is desirable to use an antibody(ies) having strongly enhanced ADCC activity and/or CDC activity.

Such an antibody having a strong cytotoxicity can be prepared, for example, from a monoclonal antibody prepared by a known method or from an already established known antibody, by increasing the cytotoxicity of the antibody by a method known in the art. In cases where an antibody that specifically recognizes the subject molecule of interest and has strong cytotoxicity is known, such an antibody may be used as an effective ingredient in the present invention. For example, WO 2010/074266 discloses an anti-CD4 antibody having a higher ADCC activity than conventional anti-CD4 antibodies.

The monoclonal antibodies include antibodies derived from non-human such as rodents, as well as chimeric antibodies, humanized antibodies (prepared by transplanting the CDR region of a non-human-derived antibody to the corresponding region of a human antibody), and human antibodies (the same antibody as an antibody produced in the body of human, which is prepared using a non-human animal or a human cell line). In cases of administration to human, a chimeric antibody with a human antibody, humanized antibody, or human antibody may be preferably used. Methods for preparing a chimeric antibody, humanized antibody, or human antibody have been established as well-known methods in the art. For example, an anti-CD4 human antibody can be prepared by immunizing, with a CD4 protein molecule, a non-human animal such as a mouse genetically modified to be capable of producing a human antibody. The gene sequence, amino acid sequence, spatial structure, and the like of CD4 have been deposited in public databases under the accession numbers of, for example, M12807 in GenBank of NCBI. The CD4 protein or an appropriate fragment thereof to be used as the immunogen can be easily prepared based on such sequence information according to well-known genetic engineering methods.

Methods for increasing the cytotoxicity of an antibody are known, and any of these methods may be used. An example of the known methods is described below.

One method for increasing the ADCC activity is the POTELLIGENT (registered trademark) technology, in which fucose (core fucose) contained in sugar chains present in the Fc region of the antibody is removed (Yamane-Ohnuki N, Satoh M, Production of therapeutic antibodies with controlled fucosylation, MAbs2009; 1: 230-236). The enzyme that adds core fucose is encoded by the gene named FucT-8 *(Fut-8).* Therefore, antibody molecules with enhanced ADCC activity can be obtained by expressing the gene encoding a recombinant antibody in *Fut-8* knockout animal cells (Yamane-Ohnuki N, et al., Establishment of FUT8 knockout Chinese hamster ovary cells: an ideal host cell line for producing completely defucosylated antibodies with enhanced antibody-dependent cellular cytotoxicity, Biotechnol Bioeng 2004; 87: 614-622). The antibody (general name, mogamulizumab) that recognizes CCR4 molecules obtained by this method has remarkably excellent cell-destroying function, and its clinical effectiveness has already been recognized. The antibody has been approved for production in Japan for application to treatment of T-cell leukemia, which develops due to infection with HTLV-1 virus (it should be noted that treatment of T cell leukemia with mogamulizumab is not based on destruction of a scaffold for HTLV-1 infection, but based on destruction of T-cell leukemia lymphoma cells expressing CCR4). A method in which fucose substrate donation is blocked is also known, but this method removes all fucose including core fucose, and hence is not specific to core fucose. Thus, the POTELLIGENT (registered trademark) technology described above is more preferred.

Another example of the method for increasing the ADCC activity is a method in which sugar chains present in the Fc region of the antibody is converted. In this method, addition of core fucose is avoided by introduction of GlcNAc in the antenna-type branched sugar chain region by GnT-III gene manipulation (M. Schuster et al., Improved effector functions of a therapeutic monoclonal Lewis Y-specific antibody by glycoform engineering, Cancer Res 2005; 65: 7934-7941). An antibody having enhanced ADCC activity prepared by such a method may also be used.

A known example of the method for enhancing the CDC activity is the COMPLEGENT (registered trademark) technology, wherein a part of isotype IgG1 is combined with the sequence of isotype IgG3 to increase the CDC activity (Natsume A, In M, Takamura H, et al. Engineered antibodies of IgG1/IgG3 mixed isotype with enhanced cytotoxic activities, Cancer Res. 2008; 68: 3863-3872).

Another known example is the AccretaMab (registered trademark) technology, wherein the POTELLIGENT (registered trademark) technology and the COMPLEGENT (registered trademark) technology described above are employed in combination to strongly increase the effector activity of an antibody (Natsume A, et al., Improving effector functions of antibodies for cancer treatment: Enhancing ADCC and CDC, Drug Des Devel Ther. 2009; 3:7-16). An antibody wherein both ADCC activity and CDC activity are increased by such a method may also be used.

Methods for evaluating the ADCC activity or the CDC activity of antibodies are known, and there are commercially available kits therefor. In the case of evaluation of the ADCC activity of an anti-CD4 antibody, the level of the ADCC activity of the anti-CD4 antibody can be evaluated by, for example, as described in the Examples below, mixing human peripheral blood mononuclear cells with the anti-CD4 antibody, allowing the reaction to proceed at 37°C for several hours, performing flow cytometry analysis to measure the ratio of CD3⁺ cells to CD8⁺ cells in the reaction solution, and then comparing the obtained measurement value with a measurement value obtained using an anti-CD4 antibody having no ADCC activity.

In addition to the above, in the present invention, as a means for specifically destroying cells having a specific molecule present in a patient's body, an antibody against the molecule or an antigen-binding fragment thereof, comprising a cytotoxic component bound thereto may be used. In cases where an antibody comprising a cytotoxic component bound thereto is used, the antibody does not need to have high cytotoxic activity, and cells containing the molecule is injured by the cytotoxic component. An antibody fragment retaining the binding capacity to the subject molecule (antigen-binding fragment), comprising a cytotoxic component bound thereto may also be used as an effective ingredient of the agent of the present invention.

In the present invention, the cytotoxic component means a substance having an activity to destroy living cells, and includes biological toxic substances, chemical substances, and radioactive substances.

The antigen-binding fragment may be any antibody fragment as long as it retains the binding capacity (antigen-antibody reactivity) to the corresponding antigen of its original antibody. Specific examples of the antigen-binding fragment include, but are not limited to, Fab, F(ab')₂, and scFv. Fab and F(ab')₂ can be obtained, as is well known, by treatment of a monoclonal antibody with a protease such as papain or pepsin. Methods for preparing scFv (single chain fragment of variable region) are also well known. For example, scFv can be obtained by extracting mRNA from a hybridoma prepared as described above, preparing single-stranded cDNA, performing PCR using primers specific to the immunoglobulin H chain and L chain to amplify the immunoglobulin H-chain gene and L-chain gene, linking these using a linker, giving an appropriate restriction enzyme site(s) to the resulting product, introducing the product into a plasmid vector, transforming *E. coli* with the resulting vector to allow expression of scFv, and then recovering the expressed scFv from *E. coli.*

The dose of the agent of the present invention is not limited as long as the subject cells can be depleted in the patient's body. The dose may vary depending on the level of the cytotoxic activity of the effective ingredient, the age and the body weight of the patient, and the like, and the dose per administration may be normally about 0.01 µg/kg to 200 mg/kg, for example, about 10 µg/kg to 150 mg/kg. The administration route may be oral or parenteral, and parenteral administration such as intravenous administration, intraarterial administration, intramuscular administration, or subcutaneous administration is preferred. The agent may be prepared as an injection or drops for use.

The administration of the agent of the present invention may be continued for several weeks to several months or longer, until the virus in the patient's body becomes undetectable. For example, at the dose described above, administration of once daily or once every several days may be continued. The dosing interval may be determined as appropriate depending on blood metabolism of the substance used as the effective ingredient. For example, in cases where an antibody is used, the administration may be carried out two or more times at an interval(s) of about one to two weeks since blood metabolism of antibodies is usually about 10 days to 14 days. If necessary, the treatment may be carried out while confirming destruction of the subject cells in the patient's body by flow cytometry analysis or the like. The cell depletion treatment is continued until the virus becomes undetectable in the patient's body. The state of cell depletion may be maintained until it is confirmed that the state where the level of the virus is below the detection limit continues for a certain period as tested by the PCR method (using the HIV RNA detection kit manufactured by Roche Diagnostics, or by the method described in the following literature: Palmer S, et al., New Real-time reverse transcriptase-initiated PCR assay with single-copy sensitivity for human immunodeficiency virus type 1 RNA in plasma, J. Clin. Microbiol., 2003; 41: 4531-4536) using a blood sample or a tissue sample of the patient.

In cases of treatment of immunodeficiency virus infection, the cells to be depleted are a part of immunocytes. Therefore, during the period of treatment for depletion of CD4-containing cells or the like in the patient's body, the health of the patient should be carefully monitored and managed by, for example, placing the patient under aseptic conditions. When the administration of the agent is stopped and the effective ingredient such as an anti-CD4 antibody is metabolized in the patient's body, CD4-containing cells derived from hematopoietic stem cells are naturally generated in the patient's body. By controlling the dose of the agent, the timing of recovery of the cells can be arbitrarily controlled. The recovery of the cells can also be confirmed by flow cytometry analysis or the like, if necessary.

### EXAMPLES

The present invention is described below by way of Examples more concretely. However, the present invention is not limited to the Examples described below.

### 1. Preparation of Anti-CD4 Antibody Having ADCC Activity

According to the method described in WO 2010/074266, an anti-CD4 humanized antibody IT1208 having enhanced ADCC activity (wherein HV2 and LV0 described in WO 2010/074266 are contained as the variable region; subtype, IgG1) was prepared. The antibody binding activity as measured using Biacore T100 was K_{D} (nM) < 0.009, which indicates high binding activity.

### 2. Measurement of ADCC Activity of Anti-CD4 Humanized Antibody IT 120 (1)

In a vial, 500 µl of PBMC derived from a healthy individual and 100 µl of a solution containing an anti-CD4 mAb (IT1208 or a control antibody) were allowed to react under the conditions of 37°C / 4 hours / 75 rpm, and the number of CD4-containing cells remaining in the reaction solution was then studied by flow cytometry analysis. As a control antibody, an antibody prepared according to the sequence of HuMax-CD4 available from GenMab, disclosed in EP1951303B1 was used. As another control, a commercially available anti-CD20 antibody (rituximab, manufactured by Zenyaku Kogyo Co., Ltd) was used. Evaluation of the activity was carried out by measurement using a flow cytometer. CD3-positive cells were detected using an FITC-labeled anti-CD3 antibody, and CD8-positive cells were detected using a phycoerythrin-labeled anti-CD8 antibody. The ratio (%) of the number of CD3-positive cells to the number of CD8-positive cells was calculated to investigate the number of CD4-positive cells (i.e., the number of CD4-positive cells is zero when the ratio defined as the number of CD3-positive cells / the number of CD8-positive cells is 100%).

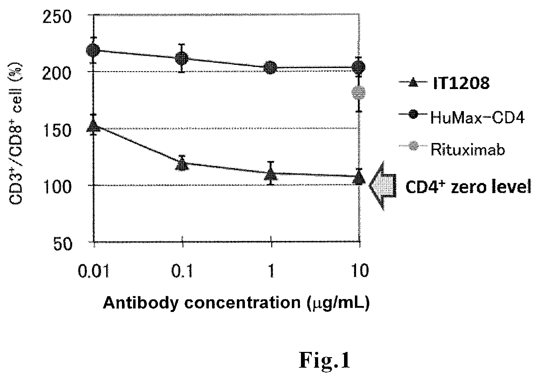

The results are shown in Fig. 1. In Humax-CD4 and rituximab, the ratio did not fall below 204% or 181%, respectively, even at an antibody concentration of 10 µg/mL, whereas, in the case of IT1208, the ratio was already 153% at 0.01 µg/mL, and 111 % or below at 1 µg/mL or more, indicating that almost all of CD4-positive cells were killed. Thus, based on comparison of the ADCC activity using NK cells in PBMCs derived from human peripheral blood and the control antibodies, IT1208 could be confirmed to have about 100 times higher ADCC activity.

### 3. Measurement of ADCC Activity of Anti-CD4 Humanized Antibody IT1208 (2)

According to the protocol for an ADCC activity assay kit, measurement of the ADCC activity of IT1208 was carried out under the following conditions. After gently mixing 12,500 PBMCs derived from a healthy individual, anti-CD4mAb (IT1208), and 75,000 ADCC Bioassay Effector cells contained in the Promega kit, the cells were cultured in a CO₂ incubator at 37°C for 6 hours. The luminescent reagent Bio-Glo reagent was added to the culture, and culturing was then continued at room temperature for 20 minutes, followed by measuring chemiluminescence using a luminescence plate reader.

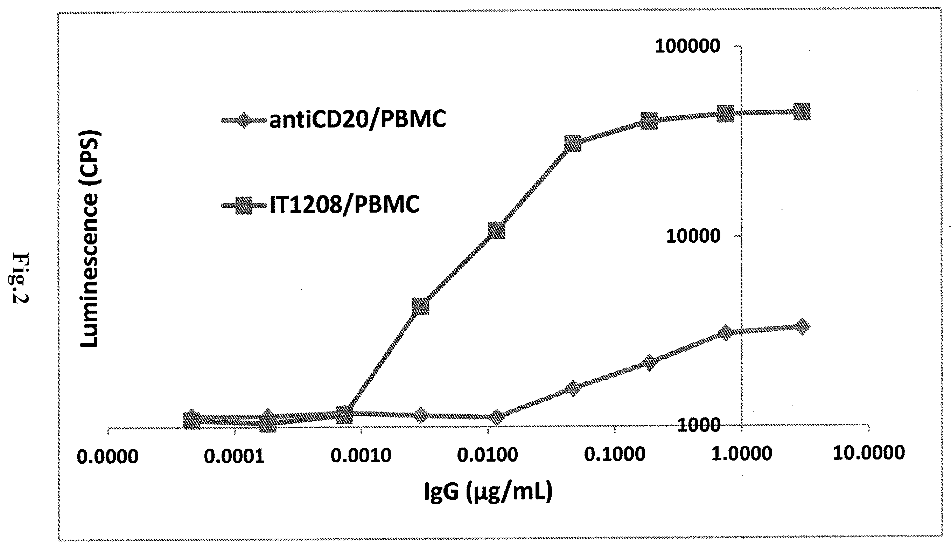

The results are shown in Fig. 2. IT1208 showed ADCC activity at 1 nM or more, and the activity then increased concentration-dependently to reach the maximum value at 50 nM. In the cases of Rituximab (antiCD20), which was used as a control antibody, the concentration at which the ADCC activity began to be found was 10 nM or more, and the concentration at which the maximum value was achieved was 1 µM or more.

### 4. Effect of ADCC-Enhanced Anti-CD4 Antibody IT1208 against HIV Infection (1)

Peripheral blood cells were collected from an HIV-infected patient receiving ART prescription (wherein the number of free HIV virus copy number in peripheral blood was lower than the detection limit; < 50 copies/ml), to provide a sample. After measuring the number of CD4 cells in the sample by flow cytometry analysis (a direct method using a fluorescent dye-labeled anti-CD4 antibody, or an indirect method using the anti-CD3 antibody and the anti-CD8 antibody, which was the same method as used in the section 2 above), the cells were stimulated with phytohemagglutinin to increase HIV virus. The increase in the copy number of the virus was confirmed by PCR, and anti-CD4 mAb (IT1208 or a control antibody) was added to the sample. The reaction was then allowed to proceed for 60 minutes, and the number of cells and the copy number of HIV RNA were measured.

### 5. Effect of ADCC-Enhanced Anti-CD4 Antibody IT1208 against HIV Infection (2)

A mouse model (Rag2-/-yc-/- (RAG-hu) mouse; Traggiai, E. et al., Development of a human adaptive immune system in cord blood cell-transplanted mice, Science, 2004; 304: 104-107) which was thought to be optimal among the humanized mouse models to be used for HIV infection was prepared, and infected with HIV virus. In this state, anti-HIV drugs (saquinavir (Chugai Pharmaceutical), lopinavir/ritonavir (Abbott Laboratories), atazanavir (Bristol-Myers-Squibb)) which were conventionally used were administered to the mouse, and the mouse was kept until HIV virus copies became undetectable in blood. The anti-CD4 mAb (IT1208) was administered to individuals in which the virus copy number in blood became less than the detection limit as observed by a conventional HIV virus detection method based on PCR, and observation was carried out.

### 6. Influence of Depletion Treatment for CD4-Containing Cells in Monkey

IT1208 prepared as described above was administered to a monkey (administration by intravenous injection at 100 mg/kg was carried out four times at one-week intervals), to cause depletion of CD4-containing cells in the body of the monkey. The monkey was then observed for half a year, but no abnormality was found.

After administering IT1208 at 40 mg/kg (four times of intravenous injection at one-week intervals) to a monkey, the numbers of CD4-containing T cells in venous blood, lymph nodes, and rectum were measured by flow cytometry analysis (a direct method using a fluorescence-labeled CD4 antibody).

The collection of the venous blood sample was carried out by collecting blood from the groin after anesthetization with ketamine + xylazine.

The lymph node sample was prepared as follows. The monkey was anesthetized with ketamine + xylazine, and the armpit was incised for about 1 to 2 cm using a knife, followed by collecting lymph nodes into a 50-ml tube. The lymph node tissue was washed twice in Hanks solution (HBSS), and then incubated in Hanks solution (HBSS) supplemented with collagenase (final concentration, 1.0 mg/ml) and DNase I (final concentration, 20 µg/ml) for 30 minutes at 37°C at 150 rpm. After passing the treated tissue through a 70-µm cell strainer, the tissue was ground, washed, and then recovered to provide a lymph node sample.

The rectal sample was prepared as follows. The monkey was anesthetized with ketamine + xylazine, and laid on its stomach on a dissection table. The anus was opened with an anoscope, and 10 pieces of the rectal tissue were collected using biopsy forceps. The rectal tissue was placed in a 50-mL tube, and an appropriate amount of MACS buffer (0.5% BSA - 2 mM EDTA in PBS(-)) was then added to the tube, followed by vortexing the tube for 10 seconds. The content of the tube was then passed through a tea strainer to recover the mucosal epithelial layer (liquid layer), and the intestinal tract was recovered into another tube. This washing treatment was repeated until the buffer became clear. The washed tissue was transferred to a conical tube, and 25 mL of a predigestion solution was added thereto. The tube was then incubated at 37°C for 30 minutes with shaking using a MACSmix (trade name) Tube Rotator (130-090-753). After vortexing the tube for 10 seconds, the content of the tube was passed through a tea strainer to recover the mucosal epithelial layer (liquid layer), and the intestinal tract was recovered into another tube. An appropriate amount of MACS buffer was added to the tube. After vortexing the tube for 10 seconds, the content of the tube was passed through a tea strainer to recover the mucosal epithelial layer (liquid layer), and the intestinal tract was recovered into another tube. This washing treatment was repeated until the buffer became clear. For removal of EDTA/DTT, about 10 mL of HBSS(+) with 10 mM HEPES (or RPMI1640) was added to the tube, and the tube was then vortexed for 10 seconds. The content of the tube was then passed through a tea strainer to recover the mucosal epithelial layer (liquid layer), and the intestinal tract was recovered into another tube. After repeating this treatment, the tissue and 2.5 mL of an enzyme mix were placed in a gentleMACS C tube, and the tissue was mildly dispersed using a gentleMACSTM Dissociator (m_brain_01). The tube was then incubated at 37°C for 30 minutes with shaking using a MACSmixTM Tube Rotator (130-090-753), and the tissue was completely dispersed using a gentleMACSTM Dissociator (m_intestine_01). The cell suspension (cells derived from the lamina propria mucosae) was collected, and diluted with an appropriate amount of MACS buffer, followed by passing the resulting dilution through a Pre-Separation Filter (70 µm, 130-095-823). MACS buffer was added to the cells, and the cells were washed (centrifugation at 4°C at 300 x g for 10 minutes). The supernatant was then discarded, and the cells were resuspended in an appropriate amount of MACS buffer, to provide a rectal sample to be subjected to flow cytometry analysis.

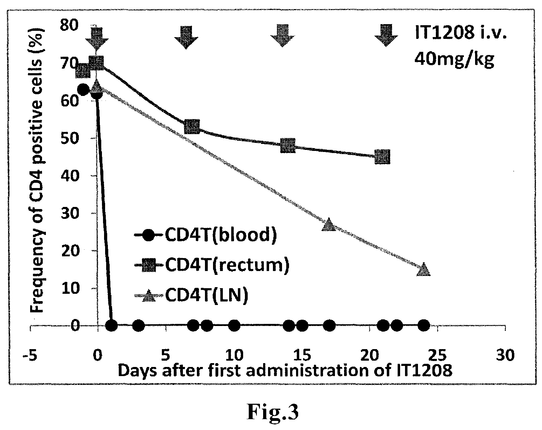

The results of the flow cytometry analysis are shown in Fig. 3. By the administration of IT1208 according to the above-described regimen, 100% of the CD4-containing T cells in the venous blood, more than 70% of those cells in the lymph nodes, and more than 40% of those cells in the rectum could be depleted. It can be thought that about one or two times of additional administration of IT1208 may allow depletion of the CD4-containing T cells in the lymph nodes. It is reported that viral recurrence after ART treatment of SIV occurs mainly from lymph nodes (Horiike et al., Virology 423 (2012) 107-118). Thus, it is thought that, for complete cure of immunodeficiency virus infection, depletion of CD4-containing cells present in the body, especially in lymph nodes, is important.

### 7. Effect of ADCC-Enhanced Anti-CD4 Antibody IT1208 against HIV Infection (3)

SIV virus was inoculated to the monkey whose CD4-containing cells were depleted by the administration of IT1208 at 100 mg/kg in the Section 6 above, and the growth of the virus was observed.

## Claims

1. A therapeutic or prophylactic agent for immunodeficiency virus infection, comprising as an effective ingredient a substance that destroys at least any of CD4 molecule-containing cells, CCR5 molecule-containing cells, and CXCR4 molecule-containing cells.

2. The therapeutic or prophylactic agent according to claim 1, comprising as an effective ingredient a substance that destroys CD4 molecule-containing cells.

3. The therapeutic or prophylactic agent according to claim 1 or 2, wherein said substance is an antibody against said molecule contained in the cells to be destroyed, which antibody has cytotoxic activity, or an antibody against said molecule contained in the cells to be destroyed or an antigen-binding fragment thereof, which antibody or antigen-binding fragment thereof comprises a cytotoxic component bound thereto.

4. The therapeutic or prophylactic agent according to claim 3, wherein said substance is an antibody against said molecule contained in the cells to be destroyed, which antibody has cytotoxic activity.

5. The therapeutic or prophylactic agent according to claim 3 or 4, wherein said cytotoxic activity is ADCC activity.

6. The therapeutic or prophylactic agent according to any one of claims 1 to 5, wherein said immunodeficiency virus is human immunodeficiency virus.

7. The therapeutic or prophylactic agent according to any one of claims 1 to 6, wherein said infection is acute infection, chronic infection, or latent infection.

8. A method for treatment or prevention of virus infection in a subject, said method comprising destroying cells which are present in the body of said subject and contain a molecule that acts as a scaffold for the virus infection.

9. The method according to claim 8, wherein said virus is an immunodeficiency virus, and wherein said method comprises destroying at least any of CD4 molecule-containing cells, CCR5 molecule-containing cells, and CXCR4 molecule-containing cells, which cells are present in the body of said subject.

10. The method according to claim 9, comprising destroying CD4 molecule-containing cells.

11. The method according to claim 9 or 10, wherein said immunodeficiency virus is human immunodeficiency virus.

12. The method according to any one of claims 8 to 11, comprising destroying said molecule-containing cells by administering to said living body an antibody against said molecule, which antibody has cytotoxic activity, or an antibody against said molecule or an antigen-binding fragment thereof, which antibody or antigen-binding fragment thereof comprises a cytotoxic component bound thereto.

13. The method according to claim 12, comprising administering to said living body an antibody against said molecule, which antibody has cytotoxic activity.

14. The method according to claim 12 or 13, wherein said cytotoxic activity is ADCC activity.

15. The method according to any one of claims 8 to 14, wherein said infection is acute infection, chronic infection, or latent infection.
